# EUROPÄISCHE PATENTSCHRIFT

(11) **EP 2 179 743 B1**
(45) Veröffentlichungstag und Bekanntmachung des Hinweises auf die Patenterteilung: **29.08.2012**
(21) Anmeldenummer: 09460028.5
(22) Anmeldetag: 13.07.2009
(51) Int. Cl.: A61K 38/18

(54) **Beschleunigungsverfahren der Entwicklung von Saugferkeln**
Method for accelerating the development of suckling piglets
Procédé d'accélération du développement de porcelets

(30) Priorität: 16.10.2008 PL 38629408
(43) Veröffentlichungstag der Anmeldung: 28.04.2010
(73) Patentinhaber: Biochefa, 41-200 Sosnowiec (PL)
(72) Erfinder: Ryszka, Florian, 41-207 Sosnowiec (PL); Dolinska, Barbara, 40-282 Katowice (PL); Leszczynska, Lucyna, 41-200 Sosnowiec (PL); Smorag, Zdzislaw, 32-083 Balice (PL); Szczesniak-Fabianczyk, Barbara, 30-108 Kraków (PL); Koska, Miroslaw, 48-250 Wolczyn (PL); Kowalski, Waldemar, 50-382 Wroclaw (PL)
(74) Vertreter: Bocionek, Karol

## Beschreibung

Die Erfindung betrifft ein Beschleunigungsverfahren von Saugferkeln, durch die Anwendung eines Prolaktinpräparates bei der Züchtung der Schweinherde.

Bei der Entwicklung der Saugferkeln ist sehr wichtig den Tieren die Bedingungen ihrer regelmäßigen Entwicklung und Erhöhung der Zahl von aufgezogenen Ferkeln zu schaffen.

Prolaktin ist unter anderem aus der WO 2010095130 A1 bekannt als Mittel zur Bereicherung von Ernährungbestandteile, Futter oder Getränke, also sogleich für Menschen als auch für Tiere, entsprechend vorbereitet in verschiedenen Dosen.

Aus WO 95/18614 ist ein Verfahren zur Behandlung der Fettleibigkeit mit Benutzung von Prolaktinmodulatoren und Diät bekannt. Das Verfahren besteht darin, weniges Essen von Kalorien mit Einnahme von Prolaktininhibitoren zu verbinden.

Ferner ist aus EP 0 315 471 A2 ein Verfahren der Unterstützung der Entwicklung von Geflügel bekannt, gemäss welchem man täglich eine Dosis von Schweineprolaktin von 1-bis 500 µg im Futter auf Kg/Masse parenteral, durch Implantat, durch Präparatinjection als auch mündlich anwendet.

Einer der Faktoren, welche die Entwicklung von Saugferkeln verursacht und ihr Wachstum beschleunigt, ist das Eiweißstoffhormon Prolaktin.

Das Prolaktin wendet man in Fällen von Laktationstörungen an, die auf Schwund oder Verminderung der Herstellung und Ausscheidung der Milch durch die Milchdrüse der Weibchen von Säugetieren im Zeitraum der Nachwuchsernährung beruhen.

Es sind Verfahren bekannt, die Entwicklung und Wachstum der Zahl von aufgezogenen Ferkel beschleunigen, und in Stimulierung der Laktation von Sauen durch Anwendung der Prolaktin aufweisenden Präparate bestehen.

Es ist auch ein Beschleunigungsverfahren der Entwicklung von Saugferkeln und Erhöhung der Zahl von aufgezogenen Ferkeln bekannt, das darin besteht, dass man den Sauen intramuskulär am Tag der Geburt von Ferkeln das Prolaktin aufweisende Präparat verabreicht.

Die Erfindung besteht darin, dass man einmalig, durch den Mund den eintägigen Ferkeln das Prolaktin aufweisende Präparat in wässerigerisotonischer Lösung in einer Menge von 0,01 µg bis 1,0 µg vorteilhaft von 0,1 µg bis 0,4 µg auf ein Kilogramm der Körpermasse anwendet.

Das Verfahren nach der Erfindung wirkt beschleunigend auf die Entwicklung von Saugferkeln, erhöhend auf ihre Körpermasse bis 40 % und vermindernd auf die Zahl, der Sterblichkeit von Tieren, und das Endergebnis am 150. Tag ist die Erreichung der Körpermasse von Schlachttieren. Beispiel 1.

Den eintägigen Ferkeln wurde einmalig durch den Mund auf ein Kilogramm der Körpermasse das Prolaktin aufweisende Präparat einer 2 µg wässerigen isotonischen Lösung verabreicht. Es ergab sich am 21. Tag der Züchtung des Ferkels, durchschnittlich ein Körpermasse-Zuwachs pro Tag auf ein Niveau von 201 Gramm und in der Kontrollgruppe von 157 Gramm.

### Beispiel 2.

Den eintägigen Ferkeln wurde einmalig durch den Mund auf ein Kilogramm der Körpermasse das Prolaktin aufweisende Präparat einer 2 µg wässerigen isotonischen Lösung verabreicht. Es ergab sich am 21. Tag der Züchtung des Ferkels durchschnittlich ein Körpermasse-Zuwachs pro Tag auf ein Niveau von 177 Gramm und in der Kontrollgruppe von 157 Gramm.

### Beispiel 3.

Den eintägigen Ferkeln wurde einmalig durch den Mund auf ein Kilogramm der Körpermasse das Prolaktin aufweisende Präparat einer 2 µg wässerigen isotonischen Lösung verabreicht. Es ergab sich am 21. Tag der Züchtung des Ferkels durchschnittlich ein Körpermasse-Zuwachs pro Tag auf ein Niveau von 180 Gramm und in der Kontrollgruppe von 181 Gramm.

### Beispiel 4

Den eintägigen Ferkeln wurde einmalig durch den Mund auf ein Kilogramm der Körpermasse das Prolaktin aufweisende Präparat einer 2 µg wässerigen- isotonischen Lösung verabreicht. Es ergab sich am 21. Tag der Züchtung des Ferkels durchschnittlich ein Körpermasse-Zuwachs pro Tag auf ein Niveau von 164 Gramm und in der Kontrollgruppe von 181 Gramm.

## Patentansprüche

1. Beschleunigungsverfahren der Entwicklung von Saugferkeln, das in Anwendung Prolaktin aufweisender Präparate besteht, **gekennzeichnet dadurch, dass** man einmalig durch den Mund den eintägigen Ferkeln das Prolaktin aufweisende Präparat in wässeriger isotonischer Lösung, in einer Menge von 001 µg bis 1,0 µg vorteilhaft von 0,1 µg bis 0,4 µg auf ein Kilogramm der Körpermasse verabreicht.

## Claims

1. Method for accelerating the development of suckling piglets based on administration of prolactin preparations, **characterized by** the fact, that one-day-old piglets are administered per orally aqueus isotonic preparation containing prolactin 0,01 µg to 1,0 µg favorably 0,1 µg to 0,4 µg per 1 kg body weight.

## Revendications

1. Procédé d'accélération du développment de porcelets pour le procédé d'accélération du développement de porcelets tétant, basé sur l'administration des préparations contenant de la prolactine, **caractérisé par** le fait d'administrer une seule fois par voie orale aux porcelets âgé d'un jour, une solution aqueuse isotonique contenant de la prolactine en quantité 0,01 µg à 1,0 µg, favorablement 0,1 µg à 0,4 µg, par kilo de poids de corps.
